# EUROPÄISCHE PATENTSCHRIFT

(11) **EP 1 458 892 B1**
(45) Veröffentlichungstag und Bekanntmachung des Hinweises auf die Patenterteilung: **03.08.2011**
(21) Anmeldenummer: 02796676.1
(22) Anmeldetag: 18.12.2002
(51) Int. Cl.: C12Q 1/68

(54) **EVANESZENZ-BASIERENDES MULTIPLEX-SEQUENZIERUNGSVERFAHREN**
EVANESCENCE-BASED MULTIPLEX SEQUENCING METHOD
PROCEDE DE SEQUEN AGE MULTIPLEX BASE SUR LE PRINCIPE DE L'EVANESCENCE

(30) Priorität: 19.12.2001 DE 10162536
(43) Veröffentlichungstag der Anmeldung: 22.09.2004
(73) Patentinhaber: Gnothis Holding SA, 1015 Ecublens (CH)
(72) Erfinder: RIGLER, Rudolf, CH-1025 St-Sulpice (CH)
(74) Vertreter: Weiss, Wolfgang
(86) Internationale Anmeldenummer: PCT/EP2002/014490
(87) Internationale Veröffentlichungsnummer: WO 2003/052137

(56) Entgegenhaltungen:
- WO-A-02/02795
- DE-A- 19 947 616
- US-A- 5 585 242
- US-A1- 2001 036 629
- US-B1- 6 225 068
- US-B1- 6 296 810
- SAUER M ET AL: "Single molecule DNA sequencing in submicrometer channels: state of the art and future prospects" JOURNAL OF BIOTECHNOLOGY, ELSEVIER SCIENCE PUBLISHERS, AMSTERDAM, NL, Bd. 86, Nr. 3, 13. April 2001 (2001-04-13), Seiten 181-201, XP004230515 ISSN: 0168-1656
- STEPHAN J ET AL: "Towards a general procedure for sequencing single DNA molecules" JOURNAL OF BIOTECHNOLOGY, ELSEVIER SCIENCE PUBLISHERS, AMSTERDAM, NL, Bd. 86, Nr. 3, 13. April 2001 (2001-04-13), Seiten 255-267, XP004230519 ISSN: 0168-1656

## Beschreibung

Die Erfindung betrifft ein Verfahren und eine Vorrichtung für eine Evaneszenz-basierende Multiplex-Sequenzierung von auf einem Träger immobilisierten Nukleinsäuremolekülen.

Die Sequenzierung des aus ca. 3 x 10⁹ Basen bestehenden humanen Genoms oder des Genoms anderer Organismen sowie die Bestimmung und der Vergleich individueller Sequenzvarianten erfordert die Bereitstellung von Sequenziermethoden, die einerseits schnell sind und andererseits routinemäßig und mit geringen Kosten eingesetzt werden können. Obwohl große Anstrengungen unternommen worden sind, um gängige Sequenziermethoden, z.B. die enzymatische Kettenabbruchmethode nach Sanger et al. (Proc. Natl. Acad. Sci. USA 74 (1977) 5463), zu beschleunigen, insbesondere durch Automatisierung (Adams et al., Automated DNA Sequencing and Analysis (1994), New York, Academic Press) können derzeit maximal nur 2.000 Basen pro Tag mit einem Sequenziergerät bestimmt werden.

Während der letzten Jahre sind neue Ansätze zur Überwindung der Beschränkungen konventioneller Sequenzierverfahren entwickelt worden, u.a. die Sequenzierung durch Rastertunnelmikroskopie (Lindsay und Phillip, Gen. Anal. Tech. Appl. 8 (1991), 8-13), durch hochparallelisierte Kapillarelektrophorese (Huang et al., Anal. Chem. 64 (1992), 2149-2154; Kambara und Takahashi, Nature 361 (1993), 565-566), durch Oligonukleotidhybridisierung (Drmanac et al., Genomics 4 (1989), 114-128; Khrapko et al., FEBS Let. 256 (1989), 118-122; Maskos und Southern, Nucleic Acids Res. 20 (1992), 1675-1678 und 1679-1684) sowie durch Matrix-unterstützte Laser-Desorptions/lonisierungs-Massenspektroskopie (Hillenkamp et al., Anal. Chem. 63 (1991), 1193A-1203A).

Ein weiterer Ansatz ist die Einzelmolekülsequenzierung (Dörre et al., Bioimaging 5 (1997), 139-152), bei der die Sequenz von Nukleinsäuren durch fortschreitenden enzymatischen Abbau von fluoreszenzmarkierten einzelsträngigen DNA-Molekülen und Nachweis der sequenziell freigesetzten Monomermoleküle in einem Mikrostrukturkanal erfolgt. Der Vorteil dieses Verfahrens besteht darin, dass nur ein einziges Molekül der Zielnukleinsäure für die Durchführung einer Sequenzbestimmung ausreicht.

Obwohl durch Anwendung der oben genannten Methoden bereits erhebliche Fortschritte erzielt wurden, besteht ein großes Bedürfnis nach weiteren Verbesserungen. Die der vorliegenden Erfindung zugrunde liegende Aufgabe bestand somit darin, ein Verfahren zur Sequenzierung von Nukleinsäuren bereitzustellen, das eine weitere Verbesserung gegenüber dem Stand der Technik darstellt und das eine parallele Bestimmung einzelner Nukleinsäuremoleküle in einem Multiplexformat ermöglicht.

In PCT/EP01/07462 wird ein Multiplex-Sequenzierungsverfahren vorgeschlagen, wobei Nukleinsäuremoleküle, die mehrere Fluoreszenzmarkierungsgruppen tragen, in immobilisierter Form auf einem Träger bereitgestellt werden, und die Basenfolge mehrere Nukelinsäuremoleküle gleichzeitig aufgrund der bei Abspaltung von Nukleotidbausteinen hervorgerufenen zeitabhängigen Änderung der Fluoreszenz der Nukleinsäuremoleküle oder/und der abgespaltenen Nukleotidbausteine bestimmt wird.

Gegenstand der vorliegenden Anmeldung ist ein Verfahren zur Sequenzierung von Nukleinsäuren, umfassend die Schritte:
(a) Bereitstellen eines zumindest teilweise optisch transparenten Trägers mit einer Vielzahl von darauf immobilisierten Nukleinsäuremolekülen, wobei die Nukleinsäuremoleküle mehrere Fluoreszenzmarkierungsgruppen tragen,
(b) fortschreitendes Abspalten einzelner Nukleotidbausteine von den immobilisierten Nukleinsäuremolekülen und
(c) gleichzeitiges Bestimmen der Basenfolge mehrerer Nukleinsäuremoleküle aufgrund der bei Abspaltung von Nukleotidbausteinen hervorgerufenen zeitabhängigen Änderung der Fluoreszenz der Nukleinsäuremoleküle oder/und der abgespaltenen Nukleotidbausteine hervorgerufenen zeitabhängigen Änderung der Fluoreszenz der Nukleinsäuremoleküle oder/und der abgespaltenen Nukleotidbausteine, wobei die Fluoreszenz durch Einstrahlen von Licht in den Träger und Erzeugen eines evaneszenten Anregungsfeldes durch interne Reflexion an der Trägeroberfläche im Bereich der immobilisierten Nukleinsäuremoleküle bewirkt wird.

Das erfindungsgemäße Verfahren ist eine trägergestützte Multiplex-Sequenziermethode, bei der eine Vielzahl von immobilisierten Nukleinsäuremolekülen gleichzeitig untersucht wird. Der für das Verfahren verwendete Träger kann ein beliebiger planarer oder strukturierter Träger sein, der zur Immobilisierung von Nukleinsäuremolekülen geeignet ist und der zumindest im Bereich der immobilisierten Nukleinsäuren eine ausreichende optische Transparenz und geeignete Oberflächenbeschaffenheit für einen Evaneszenz-basierenden Nachweis von Fluoreszenz hat. Beispiele für geeignete Trägermaterialien sind Glas, Quarz, Kunststoff oder diese Materialien enthaltende Verbundstoffe. Auch die Gestaltung des Trägers kann grundsätzlich beliebig sein, sofern ein Reaktionsraum gebildet werden kann, welcher das fortschreitende Abspalten einzelner Nukleotidbausteine von den auf dem Träger immobilisierten Nukleinsäuren in einem flüssigen Reaktionsgemisch ermöglicht.

Die Nukleinsäuremoleküle werden vorzugsweise über ihre 5'- oder 3'-Enden auf dem Träger immobilisiert, wobei sie in einzelsträngiger Form oder in doppelsträngiger Form vorliegen können. Bei doppelsträngigen Molekülen muss sichergestellt sein, dass markierte Nukleotidbausteine nur von einem einzigen Strang abgespalten werden können. Die Bindung der Nukleinsäuremoleküle an den Träger kann durch kovalente oder nicht kovalente Wechselwirkungen erfolgen. Beispielsweise kann die Bindung der Polynukleotide an den Träger durch hochaffine Wechselwirkungen zwischen den Partnern eines spezifischen Bindepaares, z.B. Biotin/Streptavidin oder Avidin, Hapten/Anti-Hapten-Antikörper, Zucker/Lectin etc., vermittelt werden. So können biotinylierte Nukleinsäuremoleküle an Streptavidin-beschichtete Träger gekoppelt werden. Alternativ können die Nukleinsäuremoleküle auch adsorptiv an den Träger gebunden werden. So kann eine Bindung von durch Einbau von Alkanthiolgruppen modifizierten Nukleinsäuremolekülen an metallische Träger, z.B. Goldträger, erfolgen. Noch eine weitere Alternative ist die kovalente Immobilisierung, wobei die Bindung der Polynukleotide über reaktive Silangruppen auf einer Silika-Oberfläche vermittelt werden kann.

An einen Träger werden mehrere, für eine Sequenzierung vorgesehene Nukleinsäuremoleküle gebunden. Vorzugsweise werden mindestens 100, besonders bevorzugt mindestens 1.000 und besonders bevorzugt mindestens 10.000 und bis zu mehr als 10⁶ Nukleinsäuremoleküle an den Träger gebunden. Die gebundenen Nukleinsäurefragmente haben eine Länge von vorzugsweise 200 bis 2.000 Nukleotide, besonders bevorzugt 400 bis 1.000 Nukleotide. Die an den Träger gebundenen Nukleinsäuremoleküle, z.B. DNA-Moleküle oder RNA-Moleküle, enthalten mehrere Fluoreszenzmarkierungsgruppen, wobei vorzugsweise mindestens 50 %, besonders bevorzugt mindestens 70 % und am meisten bevorzugt im wesentlichen alle, z.B. mindestens 90 %, der Nukleotidbausteine von einem Basentyp eine Fluoreszenzmarkierungsgruppe tragen. Derart markierte Nukleinsäuren können durch enzymatische Primerextension an einer Nukleinsäurematrize unter Verwendung einer geeigneten Polymerase, z.B. einer DNA-Polymerase wie etwa Taq-Polymerase, einer thermostabilen DNA-Polymerase von Thermococcus gorgonarius oder anderen thermostabilen Organismen (Hopfner et al., PNAS USA 96 (1999), 3600-3605) oder einer mutierten Taq-Polymerase (Patel und Loeb, PNAS USA 97 (2000), 5095-5100) unter Verwendung fluoreszenzmarkierter Nukleotidbausteine erzeugt werden.

Die markierten Nukleinsäuremoleküle können auch durch Amplifikationsreaktionen, z.B. PCR, hergestellt werden. So entstehen bei einer asymmetrischen PCR Amplifikationsprodukte, bei denen nur einziger Strang Fluroeszenzmarkierungen enthält. Derartige asymmetrische Amplifikationsprodukte können in doppelsträngiger Form sequenziert werden. Durch symmetrische PCR werden Nukleinsäurefragmente hergestellt, bei denen beide Stränge fluoreszenzmarkiert sind. Diese beiden fluoreszenzmarkierten Stränge können separiert und getrennt in einzelsträngiger Form immobilisiert werden, so daß die Sequenz eines oder beider Komplementärstränge separat bestimmt werden kann. Alternativ kann einer der beiden Stränge am 3'-Ende derart modifiziert werden, z.B. durch Einbau einer PNA-Klammer, sodass eine Abspaltung von Monomerbausteinen nicht mehr möglich ist. In diesem Fall ist eine Doppelstrangsequenzierung möglich.

Vorzugsweise tragen im Wesentlichen alle Nukleotidbausteine von mindestens zwei Basentypen, beispielsweise zwei, drei oder vier Basentypen, eine Fluoreszenzmarkierung, wobei jeder Basentyp günstigerweise eine unterschiedliche Fluoreszenzmarkierungsgruppe trägt. Wenn die Nukleinsäuremoleküle nicht vollständig markiert sind, so kann durch parallele Sequenzierung von mehreren Molekülen dennoch die Sequenz vollständig bestimmt werden.

Die Nukleinsäurematrize, deren Sequenz bestimmt werden soll, kann beispielsweise aus DNA-Matrizen wie genomischen DNA-Fragmenten, cDNA-Molekülen, Plasmiden etc., aber auch aus RNA-Matrizen wie mRNA-Molekülen ausgewählt werden.

Die Fluoreszenzmarkierungsgruppen können aus bekannten zur Markierung von Biopolymeren, z.B. Nukleinsäuren, verwendeten Fluoreszenzmarkierungsgruppen, wie etwa Fluorescein, Rhodamin, Phycoerythrin, Cy3, Cy5 oder Derivaten davon etc. ausgewählt werden.

Das erfindungsgemäße Verfahren beruht darauf, dass in Nukleinsäurestränge eingebaute Fluoreszenzmarkierungsgruppen Wechselwirkungen mit benachbarten Gruppen, beispielsweise mit chemischen Gruppen der Nukleinsäuren, insbesondere Nukleobasen wie etwa G, oder/und benachbarten Fluoreszenzmarkierungsgruppen eingehen, die zu einer Änderung der Fluoreszenz, insbesondere der Fluoreszenzintensität gegenüber den Fluoreszenzmarkierungsgruppen in "isolierter" Form aufgrund von Quench- oder/und EnergietransferVorgängen führen. Durch das Abspalten einzelner Nukleotidbausteine verändert sich die Gesamtfluoreszenz, z.B. die Fluoreszenzintensität eines immobilisierten Nukleinsäurestranges abhängig von der Abspaltung einzelner Nukleotidbausteine, d.h. abhängig von der Zeit. Diese zeitliche Änderung der Fluoreszenz kann parallel für eine Vielzahl von Nukleinsäuremolekülen erfasst und mit der Basenfolge der einzelnen Nukleinsäurestränge korreliert werden. Vorzugsweise werden solche Fluoreszenzmarkierungsgruppen verwendet, die, wenn sie in den Nukleinsäurestrang eingebaut sind, zumindest teilweise gequencht sind, so dass nach Abspaltung des die Markierungsgruppe enthaltenden Nukleotidbausteins oder eines benachbarten Bausteins, der ein Quenchen verursacht, die Fluoreszenzintensität erhöht wird.

Die Sequenzierungsreaktion des erfindungsgemäßen Verfahrens umfasst das fortschreitende Abspalten einzelner Nukleotidbausteine von den immobilisierten Nukleinsäuremolekülen. Vorzugsweise erfolgt eine enzymatische Abspaltung unter Verwendung einer Exonuklease, wobei Einzelstrang- bzw. Doppelstrang-Exonukleasen, die in 5'→3'-richtung oder 3'→5'-richtung abbauen - je nach Art der Immobilisierung der Nukleinsäurestränge auf dem Träger - eingesetzt werden können. Besonders bevorzugt werden als Exonukleasen T7 DNA-Polymerase, E.coli Exonuklease I oder E.coli Exonuklease III verwendet.

Während der fortschreitenden Abspaltung einzelner Nukleotidbausteine kann eine Änderung der Fluoreszenzintensität des immobilisierten Nukleinsäurestrangs oder/und des abgespaltenen Nukleotidbausteins aufgrund von Quench- oder Energietransfervorgängen gemessen werden. Diese zeitliche Änderung der Fluoreszenzintensität ist von der Basenfolge des untersuchten Nukleinsäurestrangs abhängig und kann daher mit der Sequenz korreliert werden. Zur vollständigen Sequenzbestimmung eines Nukleinsäurestrangs werden üblicherweise mehrere - an unterschiedlichen Basen, z.B. A, G, C und T bzw. Kombinationen von zwei verschiedenen Basen - markierte Nukleinsäurestränge vorzugsweise durch enzymatische Primerextension, wie zuvor beschrieben, erzeugt und auf dem Träger immobilisiert, wobei die Immobilisierung an statistischen Orten des Trägers oder aber auch ortsspezifisch erfolgen kann. Gegebenenfalls kann an den zu untersuchenden Nukleinsäurestrang noch ein "Sequenzidentifikator", d.h. eine markierte Nukleinsäure bekannter Sequenz, angefügt werden, z.B. durch enzymatische Reaktion mit Ligase oder/und Terminaler Transferase, sodass zu Beginn der Sequenzierung zunächst ein bekanntes Fluoreszenzmuster und anschließend erst das der unbekannten, zu untersuchenden Sequenz entsprechende Fluoreszenzmuster erhalten wird. Insgesamt werden auf einem Träger vorzugsweise 10³ bis 10⁶ Nukleinsäurestränge immobilisiert.

Um die Entfernung abgespaltener Nukleotidbausteine von den immobilisierten Nukleotidsträngen zu beschleunigen, wird im Reaktionsraum vorzugsweise ein Konvektionsfluss vom Träger weg erzeugt. Die Flussgeschwindigkeit kann dabei im Bereich von 1 bis 10 mm/s liegen.

Die Detektion umfasst ein Einstrahlen von Licht in den Träger, vorzugsweise mittels eines Lasers. Dabei können ein oder mehrere Laserstrahlen, z.B. ein aufgeweiteter Laserstrahl, mit einem Querschnitt von ca. 1-20 mm oder/und multiple Laserstrahlen verwendet werden. Durch interne Reflexion an einer oder mehreren Positionen der Trägeroberfläche im Bereich von immobilisierten Nukleinsäuremolekülen wird ein evaneszentes Anregungsfeld erzeugt, das eine Anregung der Fluoreszenzmarkierungsgruppen der auf dem Träger immobilisierten Nukleinsäuremoleküle bewirkt. Vorzugsweise ist die Reflexion an der Trägeroberfläche eine totale interne Reflexion.

Die durch evaneszente Anregung erzeugte Fluoreszenzemission mehrerer Nukleinsäurestränge kann parallel unter Verwendung einer Detektormatrix nachgewiesen werden, die beispielsweise eine elektronische Detektormatrix, z.B. eine CCD-Kamera oder eine Avalanche-Fotodiodenmatrix, umfasst. Die Detektion kann derart erfolgen, dass Fluoreszenzanregung und Detektion an allen untersuchten Nukleinsäuresträngen parallel erfolgt. Alternativ dazu kann in mehreren Schritten jeweils ein Teil der Nukleinsäurestränge untersucht werden. Vorzugsweise erfolgt der Nachweis an Fluoreszenzlicht, das im Wesentlichen orthogonal von der Trägeroberfläche abgestrahlt wird.

Noch ein weiterer Gegenstand der Erfindung ist eine Vorrichtung zur Sequenzierung von Nukleinsäuren, umfassend
(a) einen zumindest teilweise optisch transparenten Träger, umfassend eine Vielzahl von darauf immobilisierten Nukleinsäuremolekülen, wobei die Nukleinsäuremoleküle in einzelsträngiger Form vorliegen und mehrere Fluoreszenzmarkierungsgruppen tragen,
(b) einen Reaktionsraum zum fortschreitenden Abspalten einzelner Nukleotidbausteine von den immobilisierten Nukleinsäuremolekülen,
(c) Mittel zum Anregen von Fluoreszenz durch Einstrahlen von Licht in den Träger und Erzeugen eines evaneszenten Anregungsfeldes durch interne Reflexion an der Trägeroberfläche im Bereich der immobilisierten Nukleinsäuremoleküle und
(d) Mittel zum gleichzeitigen Bestimmen der Basenfolge mehrerer Nukleinsäuremoleküle aufgrund der bei Abspaltung von Nukleotidbausteinen hervorgerufenen zeitabhängigen Änderung der Fluoreszenz der Nukleinsäuremoleküle oder/und der abgespaltenen Nukleotidbausteine.

Das erfindungsgemäße Verfahren und die erfindungsgemäße Vorrichtung können beispielsweise für die Analyse von Genomen und Transkriptomen bzw. für differenzielle Analysen, z.B. Untersuchungen bezüglich des Unterschieds im Genom bzw. Transkriptom einzelner Spezies oder Organismen innerhalb einer Spezies eingesetzt werden.

Weiterhin soll die vorliegende Erfindung durch die nachfolgenden Figuren erläutert werden.

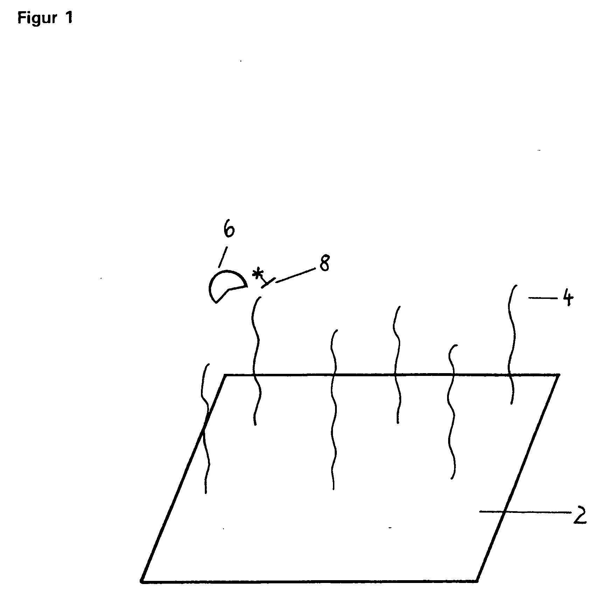

In Figur 1 ist die schematische Darstellung eines erfindungsgemäßen optisch transparenten Trägers (2) mit einer Vielzahl von daran immobilisierten einzelsträngigen markierten Nukleinsäuremolekülen (4) gezeigt. Ein Träger mit einer Fläche von 1 bis 2 cm² kann beispielsweise bis zu 10⁶ Nukleinsäurestränge enthalten.

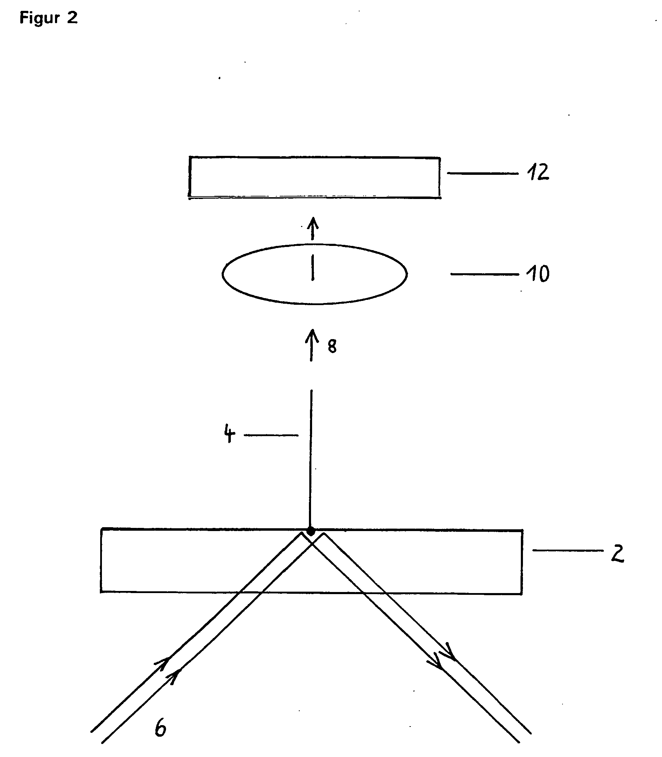

In Figur 2 ist eine erste Ausführungsform der Erfindung gezeigt, wobei in den optisch transparenten Träger (2) mit darauf immobilisierten Nukleinsäuremolekülen (4) Anregungslicht (6) von einem aufgeweiteten Laser eingestrahlt wird und nach Reflexion an der Glasoberfläche im Bereich der immobilisierten Nukleinsäuremoleküle (4) wieder aus dem Träger (2) austritt. Durch das evaneszente Anregungsfeld werden die immobilisierten Nukleinsäuremoleküle (4) zur Fluoreszenz angeregt. Das Emissionslicht (8) wird durch eine Optik (10) auf einen Detektor (12) gelenkt.

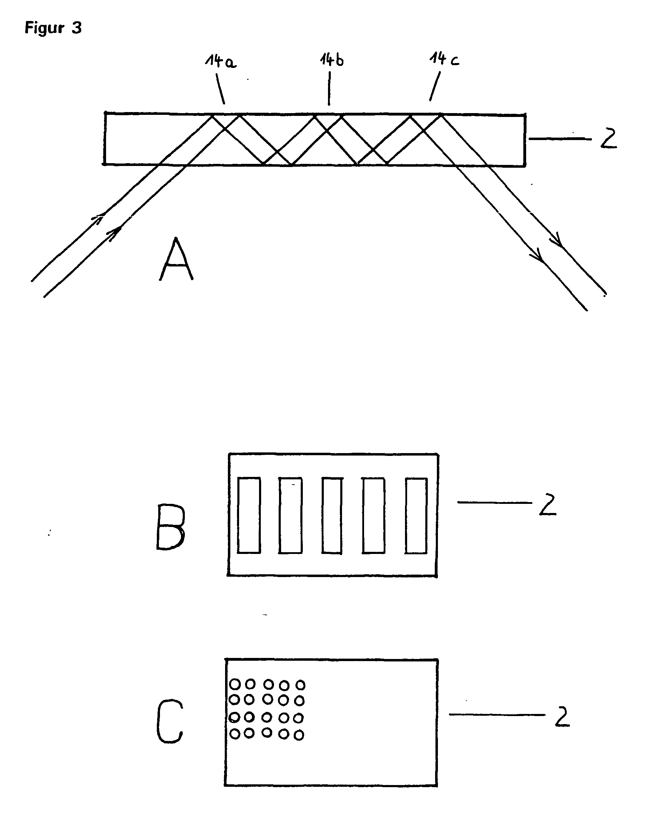

Bei der in Figur 3A gezeigten Ausführungsform werden durch multiple Reflexionen (14a, 14b, 14c) im optisch transparenten Träger (2) evaneszente Anregungsfelder erzeugt. Die evaneszenten Anregungsfelder können z.B. in Form von Streifen (Figur 3B) oder Punkten (Figur 3C) vorliegen.

Alternativ können auch mehrere Foci des Laserlichts, die durch Verwendung einer diffraktiven Optik, wie z.B. in DE 101 26 083.0 offenbart, in den Träger eingestrahlt werden.

Die durch das erfindungsgemäße Verfahren und die erfindungsgemäße Vorrichtung erreichbaren Vorteile bestehen insbesondere darin, dass Fluoreszenzanregung und -messung auf verschiedenen Seiten erfolgen. Dies bewirkt eine geringere Hintergrundstrahlung und somit eine höhere Messsensitivität.

## Patentansprüche

1. Verfahren zur Sequenzierung von Nukleinsäuren, umfassend die Schritte:
(a) Bereitstellen eines zumindest teilweise optisch transparenten Trägers mit einer Vielzahl von darauf immobilisierten Nukleinsäuremolekülen, wobei die Nukleinsäuremoleküle mehrere Fluoreszenzmarkierungsgruppen tragen,
(b) fortschreitendes Abspalten einzelner Nukleotidbausteine von den immobilisierten Nukleinsäuremolekülen, und gleichzeitiges Bestimmen der Basenfolge mehrerer Nukleinsäuremoleküle aufgrund der bei Abspaltung von Nukleotidbausteinen hervorgerufenen zeitabhängigen Änderung der Fluoreszenz der Nukleinsäuremoleküle oder/und der abgespaltenen Nukleotidbausteine,
wobei die Fluoreszenz durch Einstrahlen von Licht in den Träger und Erzeugen eines evaneszenten Anregungsfeldes durch interne Reflexion an der Trägeroberfläche im Bereich der immobilisierten Nukleinsäuremoleküle bewirkt wird.

2. Verfahren nach Anspruch 1,
**dadurch gekennzeichnet,**
**dass** man einen Träger aus Glas, Kunststoffen, Quarz oder eines mehrere dieser Materialien enthaltenden Verbundwerkstoffs verwendet.

3. Verfahren nach Anspruch 1,
**dadurch gekennzeichnet,**
**dass** eine totale interne Reflexion an der Trägeroberfläche erzeugt wird.

4. Verfahren nach einem der Ansprüche 1 bis 3,
**dadurch gekennzeichnet,**
**dass** die Nukleinsäuremoleküle derart markiert sind, dass mindestens 50 % aller Nukleotidbausteine von einem Basentyp eine Fluoreszenzmarkierungsgruppe tragen.

5. Verfahren nach Anspruch 4,
**dadurch gekennzeichnet,**
**dass** im Wesentlichen alle Nukleotidbausteine von einem Basentyp eine Fluoreszenzmarkierungsgruppe tragen.

6. Verfahren nach einem der Ansprüche 1 bis 5,
**dadurch gekennzeichnet,**
**dass** das Abspalten einzelner Nukleotidbausteine durch eine Exonuklease erfolgt.

7. Verfahren nach Anspruch 6,
**dadurch gekennzeichnet,**
**dass** T7 DNA-Polymerase, E.coli Exonuklease I oder E.coli Exonuklease III verwendet wird.

8. Verfahren nach einem der vorhergehenden Ansprüche,
**dadurch gekennzeichnet,**
**dass** das evaneszente Feld durch Einstrahlen von Licht mit einem aufgeweiteten Laser erzeugt wird.

9. Verfahren nach einem der vorhergehenden Ansprüche,
**dadurch gekennzeichnet,**
**dass** das evaneszente Feld an multiplen Bereichen des Trägers durch Einstrahlen multipler Laserstrahlen oder/und durch multiple interne Reflexionen erzeugt wird.

10. Verfahren nach einem der vorhergehenden Ansprüche,
**dadurch gekennzeichnet,**
**dass** das Bestimmen der Basenfolge eine Detektion der Fluoreszenzemission mehrerer Nukleinsäurestränge durch eine Detektionsmatrix umfasst.

11. Verfahren nach Anspruch 10,
**dadurch gekennzeichnet,**
**dass** eine CCD-Kamera oder eine Avalanche-Fotodiodenmatrix verwendet wird.

12. Verfahren nach einem der vorhergehenden Ansprüche,
**dadurch gekennzeichnet,**
**dass** Fluoreszenzanregung und -detektion an allen untersuchten Nukleinsäuresträngen parallel erfolgt.

13. Verfahren nach einem der Ansprüche 1 bis 12,
**dadurch gekennzeichnet,**
**dass** Fluoreszenzanregung und -detektion in mehreren Schritten jeweils an einen Teil der untersuchten Nukleinsäurestränge erfolgt.

14. Verfahren nach einem der vorhergehenden Ansprüche,
**dadurch gekennzeichnet,**
**dass** während der Bestimmung ein Konvektionsfluss vom Träger weg erzeugt wird.

15. Verfahren nach einem der vorhergehenden Ansprüche,
**dadurch gekennzeichnet,**
**dass** die Fluoreszenzmarkierungsgruppen, wenn sie in die Nukleinsäurestränge eingebaut sind, zumindest teilweise gequencht sind, und dass nach Abspaltung die Fluoreszenzintensität erhöht wird.

16. Vorrichtung zur Sequenzierung von Nukleinsäuren, umfassend
(a) einen zumindest teilweise optisch transparenten Träger umfassend eine Vielzahl von darauf immobilisierten Nukleinsäuremolekülen, wobei die Nukleinsäuremoleküle in einzelsträngiger Form vorliegen und mehrere Fluoreszenzmarkierungsgruppen tragen,
(b) einen Reaktionsraum zum fortschreitenden Abspalten einzelner Nukleotidbausteine von den immobilisierten Nukleinsäuremolekülen,
(c) Mittel zum Anregen von Fluoreszenz durch Einstrahlen von Licht in den Träger und Erzeugen eines evaneszenten Anregungsfeldes durch interne Reflexion an der Trägeroberfläche im Bereich der immobilisierten Nukleinsäuremoleküle und
(d) Mittel zum gleichzeitigen Bestimmen der Basenfolge mehrerer Nukleinsäuremoleküle aufgrund der bei Abspaltung von Nukleotidbausteinen hervorgerufenen zeitabhängigen Änderung der Fluoreszenz der Nukleinsäuremoleküle oder/und der abgespaltenen Nukleotidbausteine.

17. Verwendung der Vorrichtung nach Anspruch 16 in einem Verfahren nach einem der Ansprüche 1 bis 15.

## Claims

1. Method for sequencing nucleic acids comprising the steps:
(a) providing an at least partially optically transparent support with a multitude nucleic acid molecules immobilized thereon where the nucleic acid molecules carry several fluorescent marker groups,
(b) progressive cleavage of individual nucleotide building blocks from the immobilized nucleic acid molecules and simultaneous determination of the base sequence of a plurality of nucleic acid molecules based on the time-dependent change in the fluorescence of the nucleic acid molecules or/and of the cleaved nucleotide building blocks caused by the cleavage of nucleotide building blocks
wherein the fluorescence is produced by beaming light into the support and generating an evanescent excitation field by internal reflection at the support surface in the area of the immobilized nucleic acid molecules.

2. Method as claimed in claim 1,
**characterized in that**
a support made of glass, plastics, quartz or a composite containing one or more of these materials is used.

3. Method as claimed in claim 1,
**characterized in that**
a total internal reflection is generated at the support surface.

4. Method as claimed in one of the claims 1 to 3,
**characterized in that**
the nucleic acid molecules are labelled in such a manner that at least 50 % of all nucleotide building blocks of one base type carry a fluorescent marker group.

5. Method as claimed in claim 4,
**characterized in that**
essentially all nucleotide building blocks of one base type carry a fluorescent marker group.

6. Method as claimed in one of the claims 1 to 5,
**characterized in that**
individual nucleotide building blocks are cleaved by an exonuclease.

7. Method as claimed in claim 6,
**characterized in that**
T7 DNA polymerase, E. coli exonuclease I or E. coli exonuclease III is used.

8. Method as claimed in one of the previous claims,
**characterized in that**
the evanescent field is generated by beaming in light using a widened laser.

9. Method as claimed in one of the previous claims,
**characterized in that**
the evanescent field is generated at multiple areas on the support by irradiating it with multiple laser beams or/and by means of multiple internal reflections.

10. Method as claimed in one of the previous claims;
**characterized in that**
the determination of the base sequence comprises a detection of the fluorescence emission of a plurality of nucleic acid strands by a detection matrix.

11. Method as claimed in claim 10,
**characterized in that**
a CCD camera or an avalanche photodiode matrix is used.

12. Method as claimed in one of the previous claims,
**characterized in that**
the fluorescence excitation and detection of all examined nucleic acid strands is carried out in parallel.

13. Method as claimed in one of the claims 1 to 12,
**characterized in that**
the fluorescence excitation and detection is carried out in several steps and in each case on a portion of the nucleic acid strands to be examined.

14. Method as claimed in one of the previous claims,
**characterized in that**
a convection flow away from the support is generated during the determination.

15. Method as claimed in one of the previous claims,
**characterized in that**
the fluorescent marker groups are at least partially quenched when they are incorporated into the nucleic acid strands and the fluorescence intensity is increased after cleavage.

16. Device for sequencing nucleic acids comprising
(a) an at least partially optically transparent support comprising a multitude of nucleic acid molecules immobilized thereon where the nucleic acid molecules are present in a single-stranded form and carry several fluorescent marker groups,
(b) a reaction space for the progressive cleavage of individual nucleotide building blocks from the immobilized nucleic acid molecules,
(c) means for exciting fluorescence by beaming light into the support and generating an evanescent excitation field by internal reflection at the support surface in the area of the immobilized nucleic acid molecules and
(d) means for simultaneously determining the base sequence of a plurality of nucleic acid molecules based on the time-dependent change in fluorescence of the nucleic acid molecules or/and of the cleaved nucleotide building blocks caused by cleavage of nucleotide building blocks.

17. Use of the device as claimed in claim 16 in a method as claimed in one of the claims 1 to 15.

## Revendications

1. Procédé de séquençage d'acides nucléiques, comprenant les étapes de :
(a) mise à disposition d'un support au moins partiellement transparent sur le plan optique, comportant une multiplicité de molécules d'acide nucléique immobilisées sur celui-ci, les molécules d'acide nucléique portant plusieurs groupes de marquage fluorescent,
(b) clivage progressif d'éléments nucléotidiques individuels des molécules d'acide nucléique immobilisées et en même temps, détermination de la séquence de bases de plusieurs molécules d'acide nucléique sur la base de la modification de fluorescence de la molécule d'acide nucléique et/ou des éléments nucléotidiques clivés qui s'effectue en fonction du temps par clivage des élément nucléotidiques,
dans lequel la fluorescence est entraînée par un rayonnement de lumière dans les supports et la génération d'un champ d'excitation évanescent est entraîné par une réflexion interne sur la surface du support dans la zone de la molécule d'acide nucléique immobilisée.

2. Procédé selon la revendication 1, **caractérisé en ce que** l'on utilise un support en verre, en matières plastiques, en quartz ou d'un matériau composite contenant l'un de ces multiples matériaux.

3. Procédé selon la revendication 1, **caractérisé en ce qu'**une réflexion interne totale est générée sur la surface du support.

4. Procédé selon l'une des revendications 1 à 3, **caractérisé en ce que** les molécules d'acide nucléique sont marquées de telle sorte qu'au moins 50 % de tous les éléments nucléotidiques d'un type de base portent un groupe de marquage fluorescent.

5. Procédé selon la revendication 4, **caractérisé en ce qu'**essentiellement, tous les éléments nucléotidiques d'un type de base portent un groupe de marquage fluorescent.

6. Procédé selon l'une des revendications 1 à 5, **caractérisé en ce que** le clivage d'éléments nucléotidiques individuels s'effectue par une exonucléase.

7. Procédé selon la revendication 6, **caractérisé en ce que** l'on utilise la T7 ADN polymérase, l'exonucléase I de *E*. *coli* ou l'exonucléase III *de E. coli.*

8. Procédé selon l'une des revendications précédentes, **caractérisé en ce que** le champ évanescent est généré par rayonnement de lumière avec un laser élargi.

9. Procédé selon l'une des revendications précédentes, **caractérisé en ce que** le champ évanescent est généré sur des zones multiples du support par rayonnement de plusieurs rayons laser et/ou par réflexions internes multiples.

10. Procédé selon l'une des revendications précédentes, **caractérisé en ce que** la détermination de la séquence de bases comprend une détection de l'émission de fluorescence de plusieurs brins d'acide nucléique par une matrice de détection.

11. Procédé selon la revendication 10, **caractérisé en ce qu'**une caméra à dispositif de transfert de charge ou une matrice de photodiode à avalanche est utilisée.

12. Procédé selon l'une des revendications précédentes, **caractérisé en ce que** l'excitation et la détection de fluorescence s'effectuent sur tous les brins d'acide nucléique analysés.

13. Procédé selon l'une des revendications 1 à 12, **caractérisé en ce que** l'excitation et la détection de fluorescence s'effectuent en plusieurs étapes respectivement sur une partie des brins d'acide nucléique analysés.

14. Procédé selon l'une des revendications précédentes, **caractérisé en ce que**, pendant la détermination, un flux de convection est généré, s'éloignant du support.

15. Procédé selon l'une des revendications précédentes, **caractérisé en ce que** les groupes de marquage fluorescent, lorsqu'ils sont intégrés dans le brin d'acide nucléique, sont au moins partiellement inactivés et **en ce que**, après le clivage, l'intensité de fluorescence est accrue.

16. Dispositif de séquençage d'acides nucléiques, comprenant
(a) un support au moins partiellement transparent sur le plan optique, comportant une multiplicité de molécules d'acide nucléique immobilisées sur celui-ci, les molécules d'acide nucléique se présentant sous la forme de brins simples et portant plusieurs groupes de marquage fluorescent,
(b) un espace réactionnel pour le clivage progressif d'éléments nucléotidiques individuels des molécules d'acide nucléique immobilisées
(c) des moyens d'excitation de la fluorescence par rayonnement de lumière dans le support et génération d'un champ d'excitation évanescent par une réflexion interne sur la surface du support dans la zone de la molécule d'acide nucléique immobilisée et
(d) des moyens de détermination concomitante de la séquence de bases de plusieurs molécules d'acide nucléique sur la base de la modification de fluorescence de la molécule d'acide nucléique et/ou des éléments nucléotidiques clivés qui s'effectue en fonction du temps par clivage des élément nucléotidiques.

17. Utilisation du dispositif selon la revendication 16, dans un procédé selon l'une des revendications 1 à 15.
